# EUROPÄISCHE PATENTSCHRIFT

(11) **EP 1 725 516 B1**
(45) Veröffentlichungstag und Bekanntmachung des Hinweises auf die Patenterteilung: **29.08.2007**
(21) Anmeldenummer: 05707862.8
(22) Anmeldetag: 27.01.2005
(51) Int. Cl.: C07C 209/62, C07C 211/35, C07C 211/36, C07C 211/50

(54) **VERFAHREN ZUR HERSTELLUNG VON AMINEN AUS CARBODIIMIDGRUPPEN AUFWEISENDEN VERBINDUNGEN DURCH HYDROLYSE MIT WASSER**
METHOD FOR THE PRODUCTION OF AMINES FROM COMPOUNDS COMPRISING CARBODIIMIDE GROUPS, BY HYDROLYSIS WITH WATER
PROCEDE POUR PRODUIRE DES AMINES A PARTIR DE COMPOSES COMPORTANT DES GROUPES CARBODIIMIDES, PAR HYDROLYSE AU MOYEN D'EAU

(30) Priorität: 09.03.2004 DE 102004011320
(43) Veröffentlichungstag der Anmeldung: 29.11.2006
(73) Patentinhaber: Degussa GmbH, 40474 Düsseldorf (DE)
(72) Erfinder: ORSCHEL, Matthias, 48163 Münster (DE); LOMÖLDER, Rainer, 48153 Münster (DE); KOHLSTRUK, Stephan, 48249 Dülmen (DE)
(86) Internationale Anmeldenummer: PCT/EP2005/050344
(87) Internationale Veröffentlichungsnummer: WO 2005/087705

(56) Entgegenhaltungen:
- US-A- 2 938 892
- US-A- 2 941 983
- US-A- 4 927 969
- SMITH, P.A.S.: "Observations on the formation and breakdown of tetrazoles" J. AM. CHEM. SOC., Bd. 76, 1954, Seiten 436-441, XP002331591
- SMITH, P.A.S. ET AL.: "The thermal breakdown of diaryltetrazoles" J. AM. CHEM. SOC., Bd. 80, 1958, Seiten 4647-4654, XP002331592
- MOELLER F: "AMINE DURCH SPALTUNG 10. SPALTUNG MIT ALKALIEN" HOUBEN-WEYL METHODEN DER ORGANISCHEN CHEMIE, GEORGE THIEME VERLAG, STUTTGART, DE, Bd. B-XI/1, Januar 1957 (1957-01), Seiten 948-954, XP002008674 in der Anmeldung erwähnt

## Beschreibung

Die Erfindung betrifft ein ein- oder mehrstufiges Verfahren zur Herstellung von Mono-, Di- und/oder Polyaminen aus Carbodiimidgruppen und gegebenenfalls auch andere Gruppen der Isocyanatchemie aufweisenden Verbindungen durch Hydrolyse mit Wasser.

Mono-, Di- und/oder Polyamine eignen sich z. B. als Ausgangsstoffe zur Herstellung von Polyisocyanat-Polyadditionsverbindungen, als Ausgangsstoffe in Polykondensationsverfahren oder zur Herstellung von Di- oder Polyisocyanat-Verbindungen. Aliphatische Amine lassen sich durch Umsetzung von Alkylhalogeniden oder Alkoholen mit NH₃ (Ammonolyse), durch so genannte reduktive Aminierung von Ketonen oder Aldehyden, durch Aminoalkylierung (insbesondere Mannich-Reaktion), Reduktion von Amiden mit Lithiumaluminiumhydrid, katalytische Hydrierung von Nitrilen, Reduktion von Oximen mit Diboran oder von Aziden mit LiAlH4 sowie durch Hofmannschen Abbau, Curtius-Umlagerung, Ritter-Reaktion, Schmidt-Reaktion oder Gabriel-Synthese gewinnen. Die aromatischen Amine sind durch Reduktion der leicht herstellbaren Nitro-Verbindungen gut zugänglich (Ullmann's Encyclopedia of Industrial Chemistry, Wiley-VCH Verlag, 7th Edition Release 2003). Daneben lassen sich Mono-, Di- und/oder Polyamine auch durch saure oder alkalische, hydrolytische Spaltung von Urethanen, Isocyanaten und Harnstoffen synthetisieren [Houben-Weyl: Methoden der org. Chemie, 4. Auflage, Georg Thieme Verlag Stuttgart/New York (1957), 11/I, 948 ff.].

(Poly-)Carbodiimide sind bekannt und können z. B. gezielt aus substituierten Harnstoffen, Thioharnstoffen, Carbamidsäureestern, Cyanamiden, Isocyanaten, Isothiocyanaten oder anderen Carbodiimiden hergestellt werden [Houben-Weyl: Methoden der org. Chemie, 4. Auflage, Georg Thieme Verlag Stuttgart/New York (1987), E20/II, 1752; Houben-Weyl: Methoden der org. Chemie, 4. Auflage, Georg Thieme Verlag Stuttgart/New York (1983), E4, 888]. Aufgrund ihrer Reaktivität werden (Poly-)Carbodiimide z. B. als Stabilisatoren und Promotoren in der Polymerchemie oder zur Aktivierung von Carbonsäuren in der Peptidsynthese eingesetzt. Die Reaktionen von (Poly-)Carbodiimiden mit Nukleophilen, wie z. B. Wasser, Alkoholen und Carbonsäuren, sind literaturbekannt und liefern die entsprechenden (Poly-)Harnstoffe, (Poly-)Isoharnstoffe und (Poly-)Acylharnstoffe [Wagner et al., Angew. Chem. (1981), 93, 855-866; Houben-Weyl: Methoden der org. Chemie, 4. Auflage, Georg Thieme Verlag Stuttgart/New York (1987), E20/II, 1756]. Gerade die Addition von Wasser an (Poly-)Carbodiimide wurde eingehend untersucht und liefert in jedem Fall den entsprechenden Harnstoff [US 2 938 892; DE 29 41 253; Lewis et al., Chem. Eur. (2002), 8, 1934; Tordini et al., J. Phys. Chem. A (2003), 107, 1188; Kurzer et al., Chem. Rev. (1967), 67, 107].

Bis zum jetzigen Zeitpunkt war kein Verfahren zur direkten Umsetzung von Carbodiimidgruppen aufweisenden Verbindungen in die entsprechenden Amine bekannt. Aufgabe war es daher, ein solches Verfahren zu finden.

Überaschenderweise wurde nun gefunden, dass Amine direkt, ohne Isolierung der als Zwischenprodukt auftretenden Harnstoffe, aus den entsprechenden (Poly-)Carbodiimid aufweisenden Verbindungen hergestellt werden können.

Gegenstand der Erfindung ist somit ein einstufiges, kontinuierliches oder diskontinuierliches Verfahren zur Herstellung von Di- und/oder Polyaminen aus Carbodiimidgruppen aufweisenden Verbindungen durch Hydrolyse mit Wasser, wobei das entstehende Kohlendioxid aus dem Reaktionsgemisch kontinuierlich oder diskontinuierlich, unter Verwendung eines Strip-Gases entfernt wird.

Das Verfahren zur Herstellung von Mono-, Di- und/oder Polyaminen aus Carbodiimidgruppen und gegebenenfalls auch andere Gruppen der Isocyanatchemie aufweisende Verbindungen durch Hydrolyse erfolgt dadurch, dass (Poly-)Carbodiimide mit Wasser, gegebenenfalls unter Mitverwendung eines sauren oder basischen Katalysators und/oder gegebenenfalls eines Lösemittels, umgesetzt werden.

Als Carbodiimidgruppen aufweisende Verbindungen werden bevorzugt mit Gruppen der Isocyanatchemie modifizierte (Poly-)Carbodiimide eingesetzt, wie z. B. mit Urethan-, Isocyanat-, Amin-, Amid-, (Iso-)Harnstoff-, Biuret-, Isocyanurat-, Uretdion-, Guanidin-, Formamidin-, Oxamidin-, Imidazolin-, Uretonimin- und/oder Allophanat-Gruppen modifizierte aromatische, cycloaliphatische, (cyclo)aliphatische oder aliphatische (Poly-)Carbodiimide.

Bevorzugt werden die (Poly-)Carbodiimide eingesetzt, die aus (Poly-)Isocyanaten, (Poly-)Isocyanat-Derivaten oder (Poly-)Isocyanat-Homologen mit aliphatischen oder aromatischen Isocyanat-Gruppen hergestellt werden. Besonders bevorzugt werden die (Poly-)Carbodiimide eingesetzt, die aus den Polyisocyanaten, ausgewählt aus 1,4-Diisocyanatobutan, 1,6-Diisocyanatohexan (HDI), 1,12-Diisocyanatododecan, 1,4-Diisocyanatocyclohexan, 1-Isocyanato-5-isocyanatomethyl-3,3,5-trimethyl-cyclohexan (IPDI), Bis-(4-isocyanatocyclohexyl)-methan (H12MDI), 1,3-Bis-(1-isocyanato-1-methyl)-benzol (XDI), 1,3-Bis-(1-isocyanato-1-methyl-ethyl)-benzol (m-TMXDI), 2,4-Diisocyanatotoluol (TDI), Bis-(4-isocyanatophenyl)-methan (MDI), 1,6-Diisocyanato-2,2,4(2,4,4)-trimethylhexan (TMDI) und ggf. Isomeren, höheren Homologen bzw. technischen Gemischen der einzelnen Polyisocyanate, hergestellt werden.

Bevorzugt werden aus den oben genannten Carbodiimidgruppen aufweisenden Verbindungen Polyamine, ausgewählt aus 1,4-Diaminobutan, 1,6-Diaminohexan, 1,12-Diaminododecan, 1,4-Diamionocyclohexan, 1-Amino-5-aminomethyl-3,3,5-trimethyl-cyclohexan (IPDA), Bis-(4-aminocyclohexyl)-methan (H12MDA), 1,3-Bis-(1-amino-1-methyl)-benzol (XDA), 1,3-Bis-(1-amino-1-methyl-ethyl)-benzol (m-TMXDA), 2,4-Diaminotoluol (TDA), Bis-(4-aminophenyl)-methan (MDA), 1,6-Diamino-2,2,4(2,4,4)-trimethylhexan (TMDA) und ggf. Isomere, höhere Homologe bzw. technischen Gemische der einzelnen Polyamine, hergestellt.

Das Verfahren wird bevorzugt so durchgeführt, dass die Carbodiimidgruppen aufweisenden Verbindungen mit einer Menge an Wasser, die mindestens für die Hydrolyse der Carbodiimidbindungen und der gegebenenfalls mit umzusetzenden Gruppen der Isocyanatchemie ausreichend ist, bei einer Temperatur von 0 bis 400 °C und einem Druck von 0 bis 500 bar umgesetzt werden. Die gebildeten Mono-, Di- und/oder Polyamine werden durch geeignete Trennverfahren, wie Destillation, Kristallisation, Extraktion, Sorption, Permeation, Phasentrennung oder Kombinationen aus den genannten, isoliert. Die Umsetzung kann mit einem sauren oder basischen, heterogenen oder homogenen Katalysator sowie gegebenenfalls mit einem Lösemittel oder Lösemittelgemisch oder beidem erfolgen.

Die für die stöchiometrische Umsetzung benötigte Wassermenge beträgt mindestens 2 Mol Wasser pro Mol Carbodiimidgruppe und eine entsprechende Menge für die Umsetzung der gegebenenfalls zusätzlich vorhandenen Gruppen der Isocyanatchemie. Grundsätzlich ist die verwendete Wassermenge nicht limitiert. Bevorzugt wird jedoch die 2- bis 100-fache, besonders bevorzugt die 5- bis 80-fache, ganz besonders bevorzugt die 10-fache stöchiometrische Wassermenge eingesetzt.

Das erfindungsgemäße Verfahren kann ohne oder mit Lösemittel oder Lösemittelgemischen durchgeführt werden. Als Lösemittel können alle gängigen Lösemittel eingesetzt werden, bevorzugt werden Alkohole eingesetzt, besonders bevorzugt solche Alkohole, die bei der Hydrolyse von gegebenenfalls mit enthaltenen Urethangruppen gebildet werden. Das Lösemittel kann in jedem Mengenverhältnis eingesetzt werden, bevorzugt jedoch in einer ausreichenden Menge, damit das Reaktionsgemisch unter den gegebenen Reaktionsbedingungen einphasig vorliegt. Es ist allerdings auch möglich, die Umsetzung in einem zwei- oder mehrphasigen Gemisch durchzuführen und somit die anschließende Aufreinigung zu vereinfachen.

Das erfindungsgemäße Verfahren kann bei Temperaturen von 0 bis 400 °C, bevorzugt bei 150 bis 300 °C, durchgeführt werden.

Das erfindungsgemäße Verfahren kann bei einem Druck von 0 bis 500 bar, bevorzugt bei 20 bis 150 bar, durchgeführt werden. Besonders bevorzugt wird bei dem sich einstellenden Dampfdruck der Reaktionsmischung bei Reaktionstemperatur gearbeitet, welcher stark von der Zusammensetzung abhängt.

Das Einsetzen der Reaktion ist durch die Abspaltung von Kohlendioxid zu erkennen. Es ist günstig, das bei der Reaktion entstehende Kohlendioxid aus dem Reaktionsgemisch zu entfernen, damit dieses nicht für Nebenreaktionen (z. B. Carbaminsäure- bzw. Salzbildung) zur Verfügung steht. Diese Kohlendioxid-Ausschleusung aus dem Reaktionsgemisch wird kontinuierlich oder diskontinuierlich und unter Verwendung eines Strip-Gases wie z. B. Stickstoff durchgeführt.
Das erfindungsgemäße Verfahren kann kontinuierlich oder diskontinuierlich in allen gängigen Reaktorsystemen, wie z. B. in Rührkesselreaktoren, Strömungsrohrreaktoren, Wirbelschicht-reaktoren, Festbettreaktoren, Blasensäulen, Reaktivdestillationsreaktoren, Mikroreaktoren oder Kombinationen oder Kaskaden der genannten Reaktoren, erfolgen. Das Verfahren kann ein- oder mehrstufig durchgeführt werden. In einem mehrstufigen Verfahren werden Druck und Temperatur bzw. die Wasser- und/oder Katalysatormenge in den einzelnen Verfahrensschritten derart gewählt, dass das Verfahren in der ersten Stufe vom Carbodiimid bis zum Harnstoff und in der zweiten Stufe bis zum Amin durchgeführt wird. Dabei ist eine Isolierung und/oder Aufreinigung der Intermediate nicht notwendig.

Die folgenden Beispiele dienen der Erläuterung des erfindungsgemäßen Verfahrens.

### Beispiel 1 (Vergleich)

### Umsetzung von Dicyclohexylcarbodiimid zu Cyclohexylamin

500 g Dicyclohexylcarbodiimid werden in einem Autoklaven auf 190°C aufgeheizt. Anschließend werden unter Rühren aus einer auf 190 °C temperierten Vorlage 900 g Wasser zugegeben. Dabei stellt sich im Autoklaven der Dampfdruck der Reaktionsmischung ein. Während der Reaktion steigt der Druck aufgrund der Kohlendioxid-Entwicklung weiter an. Nach 4 Stunden Reaktionszeit wird der Versuch beendet und das Reaktionsgemisch gaschromatographisch untersucht. Insgesamt werden 178 g Cyclohexylamin gefunden, was einer theoretischen Ausbeute von 37 %, bezogen auf das eingesetzte Dicyclohexylcarbodiimid, entspricht.

### Beispiel 2 (Vergleich)

### Umsetzung von Dicyclohexylcarbodiimid zu Cyclohexylamin

500 g Dicyclohexylcarbodiimid werden in einem Autoklaven auf 190°C aufgeheizt. Anschließend werden unter Rühren aus einer auf 190 °C temperierten Vorlage 900 g einer wässrigen, 0,25-molaren Natriumhydroxidlösung zugegeben. Dabei stellt sich im Autoklaven der Dampfdruck der Reaktionsmischung ein. Während der Reaktion steigt der Druck aufgrund der Kohlendioxid-Entwicklung weiter an. Nach 4 Stunden Reaktionszeit wird der Versuch beendet und das Reaktionsgemisch gaschromatographisch untersucht. Insgesamt werden 298 g Cyclohexylamin gefunden, was einer theoretischen Ausbeute von 62 %, bezogen auf das eingesetzte Dicyclohexylcarbodiimid, entspricht.

### Beispiel 3 (Vergleich)

### Umsetzung von Dicyclohexylcarbodiimid zu Cyclohexylamin

Die Durchführung des Experiments erfolgt analog zum Beispiel 2, jedoch wird der Druck im Autoklaven mit Stickstoff auf 55 bar eingestellt und während der gesamten Reaktion ein Stickstoffstrom von 50 g/h durch die Reaktionsmischung geleitet, um das entstehende Kohlendioxid kontinuierlich zu entfernen.
Insgesamt werden 399 g Cyclohexylamin gefunden, was einer theoretischen Ausbeute von 83 %, bezogen auf das eingesetzte Dicyclohexylcarbodiimid, entspricht.

### Beispiel 4

### Umsetzung von

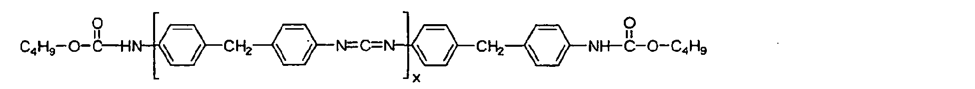

mit x = 4,7
**zu Diaminodiphenylmethan**

Das Edukt mit der Zusammensetzung 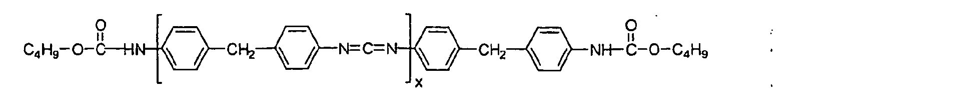 mit x = 4,7
wird nach US 2 941 983 aus Diisocyanatodiphenylmethan und n-Butanol hergestellt (x = 4,7; aus Carbodiimidgehalt und mittlerer Molmasse berechnet). 50 g dieses Polycarbodiimids werden mit 400 g n-Butanol in einem Autoklaven auf 230 °C aufgeheizt. Anschließend werden unter Rühren aus einer auf 230 °C temperierten Vorlage 100 g einer wässrigen, 0,25-molaren Natriumhydroxidlösung zugegeben und der Druck mit Stickstoff auf 55 bar eingestellt. Während der Reaktion wird ein Stickstoffstrom von 30 g/h durch die Reaktionsmischung geleitet, um das entstehende Kohlendioxid kontinuierlich zu entfernen. Nach 4 Stunden Reaktionszeit wird der Versuch beendet und das Reaktionsgemisch gaschromatographisch untersucht. Insgesamt werden 33 g Diaminodiphenylmethan gefunden, was einer theoretischen Ausbeute von 81 %, bezogen auf das eingesetzte Polycarbodiimid, entspricht.

### Beispiel 5

### Umsetzung von

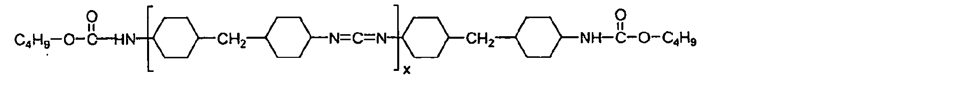

mit x = 1,1
**zu Diaminodicyclohexylmethan**

Das Edukt mit der Zusammensetzung 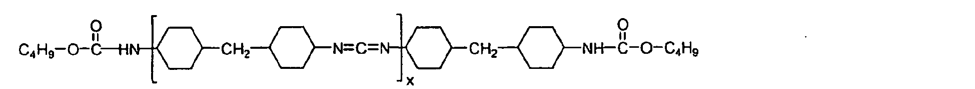 mit x = 1,1
wird analog zu Beispiel 4 aus Diisocyanatodicyclohexylmethan und n-Butanol hergestellt (x = 1,1; aus Carbodiimidgehalt und mittlerer Molmasse berechnet). 50 g dieses Polycarbodiimids werden mit 400 g n-Butanol in einem Autoklaven auf 230 °C aufgeheizt. Anschließend werden unter Rühren aus einer auf 230 °C temperierten Vorlage 100 g einer wässrigen, 0,25-molaren Natriumhydroxidlösung zugegeben und der Druck mit Stickstoff auf 55 bar eingestellt. Während der Reaktion wird ein Stickstoffstrom von 30 g/h durch die Reaktionsmischung geleitet, um das entstehende Kohlendioxid kontinuierlich zu entfernen. Nach 4 Stunden Reaktionszeit wird der Versuch beendet und das Reaktionsgemisch gaschromatographisch untersucht. Insgesamt werden 28 g Diaminodicyclohexylmethan gefunden, was einer theoretischen Ausbeute von 87 %, bezogen auf das eingesetzte Polycarbodiimid, entspricht.

## Patentansprüche

1. Einstufiges, kontinuierliches oder diskontinuierliches Verfahren zur Herstellung von Di- und/oder Polyaminen aus Carbodiimidgruppen aufweisenden Verbindungen durch Hydrolyse mit Wasser, wobei das entstehende Kohlendioxid aus dem Reaktionsgemisch kontinuierlich oder diskontinuierlich, unter Verwendung eines Strip-Gases entfernt wird.

2. Verfahren nach Anspruch 1,
**dadurch gekennzeichnet,**
**dass** als Carbodiimidgruppen aufweisende Verbindungen (Poly-)Carbodiimide eingesetzt werden, die aus (Poly-)Isocyanaten, (Poly-)Isocyanat-Derivaten oder (Poly-)Isocyanat-Homologen mit aliphatischen oder aromatischen Isocyanat-Gruppen hergestellt werden.

3. Verfahren nach mindestens einem der vorherigen Ansprüche,
**dadurch gekennzeichnet,**
**dass** als Carbodiimidgruppen aufweisenden Verbindungen (Poly-)Carbodiimide eingesetzt werden, die aus den Polyisocyanaten, ausgewählt aus 1,4-Diisocyanatobutan, 1,6-Diisocyanatohexan (HDI), 1,12-Diisocyanatododecan, 1,4-Diisocyanatocyclohexan, 1-Isocyanato-5-isocyanatomethyl-3,3,5-trimethyl-cyclohexan (IPDI), Bis-(4-isocyanatocyclohexyl)-methan (H12MDI), 1,3-Bis-(1-isocyanato-1-methyl)-benzol (XDI), 1,3-Bis-(1-isocyanato-1-methyl-ethyl)-benzol (m-TMXDI), 2,4-Diisocyanatotoluol (TDI), Bis-(4-isocyanatophenyl)-methan (MDI), 1,6-Diisocyanato-2,2,4(2,4,4)-trimethylhexan (TMDI) bzw. deren Isomeren, höheren Homologen und/oder technischen Gemischen der einzelnen Polyisocyanate, hergestellt werden.

4. Verfahren nach mindestens einem der vorherigen Ansprüche,
**dadurch gekennzeichnet,**
**dass** mit Gruppen der Isocyanatchemie modifizierte (Poly-)Carbodiimide eingesetzt werden.

5. Verfahren nach Anspruch 4,
**dadurch gekennzeichnet,**
**dass** mit Gruppen der Isocyanatchemie modifizierte (Poly-)Carbodiimide eingesetzt werden, ausgewählt aus mit Urethan-, Isocyanat-, Amin-, Amid-, (Iso-)Harnstoff-, Biuret-, Isocyanurat-, Uretdion-, Guanidin-, Formamidin-, Oxamidin-, Imidazolin-, Uretonimin- und/oder Allophanat-Gruppen modifizierte aromatische, cycloaliphatische, (cyclo)aliphatische oder aliphatische (Poly-)Carbodiimide.

6. Verfahren nach mindestens einem der vorherigen Ansprüche,
**dadurch gekennzeichnet,**
**dass** mit einer Menge an Wasser, die mindestens für die Hydrolyse der Carbodiimidbindungen und der gegebenenfalls mit umzusetzenden Gruppen der Isocyanatchemie ausreichend ist, umgesetzt wird.

7. Verfahren nach mindestens einem der vorherigen Ansprüche,
**dadurch gekennzeichnet,**
**dass** die eingesetzte Wassermenge mindestens 2 Mol Wasser pro Mol Carbodiimidgruppe und eine entsprechende Menge für die Umsetzung der gegebenenfalls zusätzlich vorhandenen Gruppen der Isocyanatchemie beträgt.

8. Verfahren nach Anspruch 7,
**dadurch gekennzeichnet,**
**dass** die eingesetzte Wassermenge die 5- bis 100fache stöchiometrische Menge, bevorzugt die 5- bis 80fache, besonders bevorzugt die 10fache stöchiometrische Menge, bezogen auf die zur Umsetzung der Carbodiimidgruppen und der gegebenenfalls zusätzlich vorhandenen Gruppen der Isocyanatchemie benötigten stöchiometrischen Wassermenge, beträgt.

9. Verfahren nach mindestens einem der vorherigen Ansprüche,
**dadurch gekennzeichnet,**
**dass** mit einem sauren oder basischen, heterogenen oder homogenen Katalysator oder Mischungen aus sauren oder basischen, heterogenen oder homogenen Katalysatoren umgesetzt wird.

10. Verfahren nach mindestens einem der vorherigen Ansprüche,
**dadurch gekennzeichnet,**
**dass** bei einer Temperatur von 0 bis 400 °C umgesetzt wird.

11. Verfahren nach Anspruch 10,
**dadurch gekennzeichnet,**
**dass** das Verfahren bei Temperaturen von 150 bis 300 °C durchgeführt wird.

12. Verfahren nach mindestens einem der vorherigen Ansprüche,
**dadurch gekennzeichnet,**
**dass** bei einem Druck von 0 bis 500 bar umgesetzt wird.

13. Verfahren nach Anspruch 12,
**dadurch gekennzeichnet,**
**dass** das Verfahren bei einem Druck von 20 bis 150 bar durchgeführt wird.

14. Verfahren nach mindestens einem der vorherigen Ansprüche;
**dadurch gekennzeichnet,**
**dass** die gebildeten Mono-, Di- und/oder Polyamine durch Trennverfahren ausgewählt aus Destillation, Kristallisation, Extraktion, Sorption, Permeation, Phasentrennung oder Kombinationen dieser aufgearbeitet werden.

15. Verfahren nach mindestens einem der vorherigen Ansprüche,
**dadurch gekennzeichnet,**
**dass** mit oder ohne Lösemittel umgesetzt wird.

16. Verfahren nach Anspruch 15,
**dadurch gekennzeichnet,**
**dass** als Lösemittel oder Lösemittelgemisch Alkohole eingesetzt werden, bevorzugt solche Alkohole, die bei der Hydrolyse von gegebenenfalls mit enthaltenen Urethangruppen gebildet werden.

17. Verfahren nach mindestens einem der vorherigen Ansprüche,
**dadurch gekennzeichnet,**
**dass** bei dem sich einstellenden Dampfdruck der Reaktionsmischung bei Reaktionstemperatur gearbeitet wird.

18. Verfahren nach mindestens einem der vorherigen Ansprüche,
**dadurch gekennzeichnet,**
**dass** das bei dem Verfahren entstehende Kohlendioxid aus dem Reaktionsgemisch kontinuierlich oder diskontinuierlich, unter Verwendung von Stickstoff entfernt wird.

19. Verfahren nach mindestens einem der vorherigen Ansprüche,
**dadurch gekennzeichnet,**
**dass** das Verfahren kontinuierlich oder diskontinuierlich in Reaktorsystemen, ausgewählt aus Rührkesselreaktoren, Strömungsrohrreaktoren, Wirbelschicht-Reaktoren, Festbettreaktoren, Blasensäulen, Reaktivdestillationsreaktoren, Mikroreaktoren oder Kombinationen oder Kaskaden der genannten Reaktoren, durchgeführt wird.

20. Verfahren nach mindestens einem der vorherigen Ansprüche,
**dadurch gekennzeichnet,**
**dass** Polyamine, ausgewählt aus 1,4-Diaminobutan, 1,6-Diaminohexan, 1,12-Diaminododecan, 1,4-Diamionocyclohexan, 1-Amino-5-aminomethyl-3,3,5-trimethylcyclohexan (IPDA), Bis-(4-aminocyclohexyl)-methan (H12MDA), 1,3-Bis-(1-amino-1-methyl)-benzol (XDA), 1,3-Bis-(1-amino-1-methyl-ethyl)-benzol (m-TMXDA), 2,4-Diaminotoluol (TDA), Bis-(4-aminophenyl)-methan (MDA), 1,6-Diamino-2,2,4(2,4,4)-trimethylhexan (TMDA) und ggf. Isomere, höhere Homologe bzw. technischen Gemische der einzelnen Polyamine, hergestellt werden.

## Claims

1. A one-stage, continuous or batchwise process for preparing di- and/or polyamines from compounds having carbodiimide groups by hydrolysis with water, the carbon dioxide formed being removed from the reaction mixture continuously or discontinuously, using a stripping gas.

2. A process according to claim 1,
**characterized in that**
the compounds having carbodiimide groups which are used are (poly)carbodiimides which are prepared from (poly)isocyanates, (poly)isocyanate derivatives or (poly)isocyanate homologues having aliphatic or aromatic isocyanate groups.

3. A process according to at least one of the preceding claims,
**characterized in that**
the compounds having carbodiimide groups which are used are (poly)carbodiimides which are prepared from the polyisocyanates selected from 1,4-diisocyanatobutane, 1,6-diisocyanatohexane (HDI), 1,12-diisocyanatododecane, 1,4-diisocyanatocyclohexane, 1-isocyanato-5-isocyanatomethyl-3,3,5-trimethylcyclohexane (IPDI), bis(4-isocyanatocyclohexyl)methane (H12MDI), 1,3-bis(1-isocyanato-1-methyl)benzene (XDI), 1,3-bis(1-isocyanato-1-methylethyl)benzene (m-TMXDI), 2,4-diisocyanatotoluene (TDI), bis(4-isocyanatophenyl)methane (MDI), 1,6-diisocyanato-2,2,4 (2,4,4)-trimethylhexane (TMDI), or isomers thereof, higher homologues thereof and/or technical-grade mixtures of the individual polyisocyanates.

4. A process according to at least one of the preceding claims,
**characterized in that**
(poly)carbodiimides are used which have been modified with groups of isocyanate chemistry.

5. A process according to claim 4,
**characterized in that**
(poly)carbodiimides are used which have been modified with groups of isocyanate chemistry and are selected from aromatic, cycloaliphatic, (cyclo)aliphatic or aliphatic (poly)carbodiimides and have been modified with urethane, isocyanate, amine, amide, (iso)urea, biuret, isocyanurate, uretdione, guanidine, formamidine, oxamidine, imidazoline, uretonimine and/or allophanate groups.

6. A process according to at least one of the preceding claims,
**characterized in that**
reaction is effected with an amount of water which is sufficient at least for the hydrolysis of the carbodiimide bonds and of any groups of isocyanate chemistry which are also to be converted.

7. A process according to at least one of the preceding claims,
**characterized in that**
the amount of water used is at least 2 mol of water per mole of carbodiimide group and a corresponding amount for the conversion of any groups of isocyanate chemistry which are additionally present.

8. A process according to claim 7,
**characterized in that**
the amount of water used is 5 to 100 times the stoichiometric amount, preferably 5 to 80 times, more preferably 10 times the stoichiometric amount, based on the stoichiometric amount of water required to convert the carbodiimide groups and any groups of isocyanate chemistry additionally present.

9. A process according to at least one of the preceding claims,
**characterized in that**
reaction is effected with an acidic or basic, heterogeneous or homogeneous catalyst or mixtures of acidic or basic, heterogeneous or homogeneous catalysts.

10. A process according to at least one of the preceding claims,
**characterized in that**
reaction is effected at a temperature of 0 to 400°C.

11. A process according to claim 10,
**characterized in that**
the process is performed at temperatures of 150 to 300°C.

12. A process according to at least one of the preceding claims,
**characterized in that**
reaction is effected at a pressure of 0 to 500 bar.

13. A process according to claim 12,
**characterized in that**
the process is performed at a pressure of 20 to 150 bar.

14. A process according to at least one of the preceding claims,
**characterized in that**
the mono-, di- and/or polyamines formed are worked up by separation processes selected from distillation, crystallization, extraction, sorption, permeation, phase separation or combinations thereof.

15. A process according to at least one of the preceding claims,
**characterized in that**
reaction is effected with or without solvent.

16. A process according to claim 15,
**characterized in that**
the solvent or solvent mixture used is alcohols, preferably those alcohols which are formed in the hydrolysis of any urethane groups also present.

17. A process according to at least one of the preceding claims,
**characterized in that**
the working pressure is the vapour pressure of the reaction mixture which is established at reaction temperature.

18. A process according to at least one of the preceding claims,
**characterized in that**
carbon dioxide formed in the process is removed from the reaction mixture continuously or discontinuously, using nitrogen.

19. A process according to at least one of the preceding claims,
**characterized in that**
the process is carried out continuously or batchwise in reactor systems selected from stirred tank reactors, flow tube reactors, fluidized bed reactors, fixed bed reactors, bubble columns, reactive distillation reactors, microreactors or combinations or batteries of the reactors mentioned.

20. A process according to at least one of the preceding claims,
**characterized in that**
polyamines selected from 1,4-diaminobutane, 1,6-diaminohexane, 1,12-diaminododecane, 1,4-diamionocyclohexane, 1-amino-5-aminomethyl-3,3,5-trimethylcyclohexane (IPDA), bis(4-aminocyclohexyl)methane (H12MDA), 1,3-bis(1-amino-1-methyl)benzene (XDA), 1,3-bis(1-amino-1-methylethyl)benzene (m-TMXDA), 2,4-diaminotoluene (TDA), bis(4-aminophenyl)methane (MDA), 1,6-diamino-2,2,4(2,4,4)-trimethylhexane (TMDA) and where appropriate isomers, higher homologues and technical-grade mixtures of the individual polyamines are prepared.

## Revendications

1. Procédé continu ou discontinu en une étape pour la préparation de di- et/ou polyamines à partir de composés présentant des groupes carbodiimide par hydrolyse avec de l'eau, le dioxyde de carbone formé étant éliminé de manière continue ou discontinue du mélange réactionnel en utilisant un gaz de stripping.

2. Procédé selon la revendication 1,
**caractérisé en ce**
**que** l'on utilise comme composés présentant des groupes carbodiimide des (poly)carbodiimides qui sont préparés à partir de (poly)isocyanates, de dérivés de (poly)isocyanate ou d'homologues de (poly)isocyanate avec des groupes isocyanate aliphatiques ou aromatiques.

3. Procédé selon au moins l'une des revendications précédentes,
**caractérisé en ce**
**que** l'on utilise comme composés présentant des groupes carbodiimide des (poly)carbodiimides qui sont préparés à partir des polyisocyanates sélectionnés parmi le 1,4-diisocyanatobutane, le 1,6-diisocyanatohexane (HDI), le 1,12-diisocyanatododécane, le 1,4-diisocyanatocyclohexane, le 1-isocyanato-5-isocyanatométhyl-3,3,5-triméthylcyclohexane (IPDI), le bis(4-isocyanatocyclohexyl)-méthane (H12MDI), le 1,3-bis(1-isocyanato-1-méthyl)-benzène (XDI), le 1,3-bis(1-isocyanato-1-méthyléthyl)-benzène (m-TMXDI), le 2,4-diisocyanatotoluène (TDI), le bis-(4-isocyanatophényl)-méthane (MDI), le 1,6-diisocyanato-2,2,4(2,4,4)-triméthylhexane (TMDI) ou leurs isomères, homologues supérieurs et/ou des mélanges techniques des différents polyisocyanates.

4. Procédé selon au moins l'une des revendications précédentes,
**caractérisé en ce**
**que** des (poly)carbodiimides modifiés avec des groupes de la chimie des isocyanates sont utilisés.

5. Procédé selon la revendication 4,
**caractérisé en ce**
**que** l'on utilise des (poly)carbodiimides modifiés avec des groupes de la chimie des isocyanates, sélectionnés dans le groupe des (poly)carbodiimides aromatiques, cycloaliphatiques, (cyclo) aliphatiques ou aliphatiques modifiés avec des groupes uréthane, isocyanate, amine, amide, (iso)urée, biuret, isocyanurate, uretdione, guanidine, formamidine, oxamidine, imidazoline, urétonimine et/ou allophanate.

6. Procédé selon au moins l'une des revendications précédentes,
**caractérisé en ce que** la mise en réaction se déroule avec une quantité d'eau qui suffit au moins pour l'hydrolyse des liaisons carbodiimide et des groupes de la chimie des isocyanates à mettre éventuellement en réaction.

7. Procédé selon au moins l'une des revendications précédentes,
**caractérisé en ce**
**que** la quantité d'eau utilisée s'élève à au moins 2 moles d'eau par mole de groupe carbodiimide et une quantité correspondante pour la mise en réaction des groupes éventuellement présents en plus de la chimie des isocyanates.

8. Procédé selon la revendication 7,
**caractérisé en ce**
**que** la quantité d'eau utilisée s'élève à 5 à 100 fois la quantité stoechiométrique, de préférence 5 à 80 fois, tout particulièrement 10 fois la quantité stoechiométrique par rapport à la quantité d'eau stoechiométrique nécessaire pour la mise en réaction des groupes carbodiimide et des groupes présents éventuellement en plus de la chimie des isocyanates.

9. Procédé selon au moins l'une des revendications précédentes,
**caractérisé en ce**
**que** l'on fait réagir en utilisant un catalyseur homogène ou hétérogène, acide ou basique ou des mélanges de catalyseurs hétérogènes ou homogènes acides ou basiques.

10. Procédé selon au moins l'une des revendications précédentes,
**caractérisé en ce**
**que** la réaction se déroule à une température de 0 à 400°C.

11. Procédé selon la revendication 10,
**caractérisé en ce**
**que** le procédé se déroule à des températures de 150 à 300°C.

12. Procédé selon au moins l'une des revendications précédentes,
**caractérisé en ce**
**que** la réaction se déroule à une pression de 0 à 500 bars.

13. Procédé selon la revendication 12,
**caractérisé en ce**
**que** le procédé se déroule à une pression de 20 à 150 bars.

14. Procédé selon au moins l'une des revendications précédentes,
**caractérisé en ce**
**que** les mono-, di- et/ou polyamines formées sont traitées par un procédé de séparation sélectionné parmi la distillation, la cristallisation, l'extraction, la sorption, la perméation, la séparation de phases ou des combinaisons de celles-ci.

15. Procédé selon au moins l'une des revendications précédentes,
**caractérisé en ce**
**que** la réaction se déroule avec ou sans solvant.

16. Procédé selon la revendication 15,
**caractérisé en ce**
**que** l'on utilise comme solvant ou mélange de solvants des alcools, de préférence des alcools qui sont formés éventuellement lors de l'hydrolyse de groupes uréthanne contenus.

17. Procédé selon au moins l'une des revendications précédentes,
**caractérisé en ce**
**que** l'on travaille à la température de réaction lorsque la pression de vapeur du mélange réactionnel s'installe.

18. Procédé selon au moins l'une des revendications précédentes,
**caractérisé en ce que** le dioxyde de carbone formé lors du procédé est éliminé de manière continue ou discontinue du mélange réactionnel en utilisant de l'azote.

19. Procédé selon au moins l'une des revendications précédentes,
**caractérisé en ce**
**que** le procédé se déroule de manière continue ou discontinue dans des systèmes de réacteurs sélectionnés à partir de réacteurs agités, réacteurs à écoulement, réacteurs à lit fluidisé, réacteurs à lit fixe, colonnes à bulles, réacteurs de distillation réactive, microréacteurs ou des combinaisons ou cascades desdits réacteurs.

20. Procédé selon au moins l'une des revendications précédentes,
**caractérisé en ce**
**que** l'on prépare des polyamines sélectionnées parmi le 1,4-diaminobutane, le 1,6-diaminohexane, le 1,12-diaminododécane, le 1,4-diaminocyclohexane, le 1-amino-5-aminométhyl-3,3,5-triméthylcyclohexane (IPDA), le bis(4-aminocyclohexyl)-méthane (H12MDA), le 1,3-bis(1-amino-1-méthyl)-benzène (XDA), le 1,3-bis(1-amino-1-méthyléthyl)-benzène (m-TMXDA), le 2,4-diaminotoluène (TDA), le bis(4-aminophényl)-méthane (MDA), le 1,6-diamino-2,2,4(2,4,4)-triméthylhexane (TMDA) et, éventuellement, des isomères, des homologues supérieurs ou des mélanges techniques des différentes polyamines.
